(19) Europäisches Patentamt
European Patent Office
Office européen des brevets

(11) **EP 4 122 480 B1**

(12) **EUROPEAN PATENT SPECIFICATION**

(45) Date of publication and mention of the grant of the patent:
**19.08.2026 Bulletin 2026/34**

(21) Application number: **21771258.7**

(22) Date of filing: **17.03.2021**

(51) International Patent Classification (IPC):
***A61K 36/481*** *(2006.01)* ***A61K 31/7004*** *(2006.01)*
***A61P 35/00*** *(2006.01)* ***A61K 31/44*** *(2006.01)*
***A61K 31/502*** *(2006.01)* ***A61K 31/5377*** *(2006.01)*

(52) Cooperative Patent Classification (CPC):
(C-Sets available)
**A61P 35/00; A61K 31/44; A61K 31/502; A61K 31/517; A61K 31/7004; A61K 31/715; A61K 36/481** (Cont.)

(86) International application number:
**PCT/CN2021/081377**

(87) International publication number:
**WO 2021/185292 (23.09.2021 Gazette 2021/38)**

(54) **USE OF ASTRAGALUS MEDICINAL COMPOSITION FOR PREPARING DRUG FOR ENHANCING CANCER THERAPY**

VERWENDUNG EINER MEDIZINISCHEN ASTRAGALUS-ZUSAMMENSETZUNG ZUR HERSTELLUNG EINES ARZNEIMITTELS ZUR VERBESSERUNG DER KREBSTHERAPIE

UTILISATION D'UNE COMPOSITION MÉDICINALE D'ASTRAGALE POUR PRÉPARER UN MÉDICAMENT POUR AMÉLIORER UNE THÉRAPIE ANTICANCÉREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2020 US 202062990650 P**

(43) Date of publication of application:
**25.01.2023 Bulletin 2023/04**

(73) Proprietor: **PhytoHealth Corporation**
**Taipei, Taiwan 105 (TW)**

(72) Inventors:
- **LEE, I-Lin**
  **Songshan Taipei, Taiwan 105 (TW)**
- **CHENG, Chien-Hsin**
  **Songshan Taipei, Taiwan 105 (TW)**

(74) Representative: **karo IP**
**Patentanwälte PartG mbB**
**Steinstraße 16-18**
**40212 Düsseldorf (DE)**

(56) References cited:
**CN-A- 1 557 826**
**CN-A- 101 347 445**
**CN-A- 102 443 072**
**CN-A- 103 830 262**
**CN-A- 109 512 857**
**CN-A- 110 193 021**
**DE-U1- 202020 101 993**
**JP-U- 3 228 581**

- **WANG JIA ET AL: "Extraction, Structure, and Pharmacological Activities of Astragalus Polysaccharides", APPLIED SCIENCES, vol. 9, no. 1, 31 December 2018 (2018-12-31), pages 122, XP093057840, DOI: 10.3390/app9010122**
- **GONZALEZ PABLO SIERRA ET AL: "Mannose impairs tumour growth and enhances chemotherapy", NATURE, NATURE PUBLISHING GROUP UK, LONDON, vol. 563, no. 7733, 21 November 2018 (2018-11-21), pages 719 - 723, XP036647883, ISSN: 0028-0836, [retrieved on 20181121], DOI: 10.1038/S41586-018-0729-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ANONYMOUS: "Abstract LB-051: An Astragalus-based Chinese herbal medicine extraction inhibits cancer stem cell growth and sensitizes of drug-resistant human non-small cell lung cancer cells for targeted therapy | Cancer Research | American Association for Cancer Research", 1 January 2018 (2018-01-01), XP093058821, Retrieved from the Internet <URL:https://aacrjournals.org/cancerres/article/78/13_Supplement/LB-051/631057/Abstract-LB-051-An-Astragalus-based-Chinese-herbal> [retrieved on 20230628]**
- **TIAN QING-E ET AL: "Astragalus polysaccharides can regulate cytokine and P-glycoprotein expression in H22 tumor-bearing mice", vol. 18, no. 47, 1 January 2012 (2012-01-01), CN, pages 7079, XP093058671, ISSN: 1007-9327, Retrieved from the Internet <URL:https://www.ncbi.nlm.nih.gov/pmc/articles/PMC3531697/pdf/WJG-18-7079.pdf> DOI: 10.3748/wjg.v18.i47.7079**
- **SONG JIE ET AL: "Astragalus Polysaccharide Promotes Adriamycin-Induced Apoptosis in Gastric Cancer Cells", vol. Volume 12, 1 January 2020 (2020-01-01), pages 2405 - 2414, XP093058666, Retrieved from the Internet <URL:https://www.ncbi.nlm.nih.gov/pmc/articles/PMC7131994/pdf/cmar-12-2405.pdf> DOI: 10.2147/CMAR.S237146**
- **PHARM TROP J ET AL: "Recent perspectives on chemosensitizing effects of Astragalus membranaceus-derived components: A mini review", TROPICAL JOURNAL OF PHARMACEUTICAL RESEARCH NOVEMBER JOURNAL CITATION REPORTS/SCIENCE EDITION, 1 November 2019 (2019-11-01), pages 2455, XP093058823, Retrieved from the Internet <URL:www.ajol.info> [retrieved on 20230628], DOI: 10.4314/tjpr.v18i11.32**
- **DENG XIAOXIA , LI QINGSONG , CHEN ZHONG , CHEN JIEYING , WANG YAO , LIN SEQI: "Advances in Antitumor Mechanisms of Radix Astragali", ZHONGYAO XINYAO YU LINGCHUANG YAOLI - TRADITIONAL CHINESE DRUG RESEARCH & CLINICAL PHARMACOLOGY, vol. 27, no. 2, 25 March 2016 (2016-03-25), pages 307 - 312, XP055851378, ISSN: 1003-9783, DOI: 10.3969/j.issn.1003-9783.2016.02.032**
- **SONG MENG-MENG , CHEN ZHE-WEN , LI YE , SONG CHUN-HUA , SHI HAN-PING , MIAO MINIG-YONG: "Advances in Anti-Tumor Effect of Mannose", ELECTRONIC JOURNAL OF METABOLISM AND NUTRITION OF CANCER, vol. 6, no. 3, 9 September 2019 (2019-09-09), pages 283 - 286, XP055851380, DOI: 10.16689/j.cnki.cn11-9349/r.2019.03.003**



(52) Cooperative Patent Classification (CPC): (Cont.)

C-Sets
**A61K 31/7004, A61K 2300/00;**
**A61K 31/715, A61K 2300/00;**
**A61K 36/481, A61K 2300/00**

## Description

### FIELD OF THE INVENTION

The invention is as defined in claim 1, and relates to an astragalus pharmaceutical composition for use in a method of enhancing the treatment of a chemotherapy drug in a cancer on inhibiting the viability of a cancer cell.

### BACKGROUND OF THE INVENTION

The astragalus is Astragalus mongholicus Bunge or Astragalus mongholicus, to be specific, the astragalus refers to the dry root of *Astragalus membranaceus* Bge. var. *mongholicus* (Bge.) Hsiao or *A. membranaceus* (Fisch.) Bge. According to the record of traditional pharmacy, the astragalus is used for treating chronic nephritis, proteinuria, myositis, hypertension, coronary heart disease, cerebral infarction, peptic ulcer (duodenal and gastric ulcers), the kidney disease and diabetes. Additionally, a few kinds of r-APS can be used for treating tumors. Some studies show that the tumor treatment mechanisms include adjusting immune response (Lau, B.H.S., Ruckle, H.C. Botolazzo, T.) and "using Chinese herbal medicine to inhibit the growth of mouse's renal-cell carcinoma" of Lui, P.D.

The composition of astragalus has been analyzed in recent years, including monosaccharide, polysaccharide, flavone, amino acid and trace element. The content of polysaccharide is the highest and the treatment effect of polysaccharide on diseases has been proved, especially on the cancer related diseases, e.g. tumors (American Patent No.5,268,467; "recent development of antineoplastic agent of science of Chinese herbal medicine" of Tang, W. Hemm, I. and Bertram, B.), Part II, high molecular weight compounds.

For rapid growth, the malignant tumor consumes more glucose than normal healthy tissues, it is hard to control internal glucose content only by diet. The Cancer Research UK Beatson Institute indicates that "the tumor needs a lot of glucose for growth, so the cancer development can be slowed down by restricting the available glucose for tumor. The question is that normal tissues need glucose, too. Therefore, we cannot remove glucose thoroughly. It was found in research that a certain dose of mannose could block a considerable amount of glucose, so as to slow down the mouse's tumor growth, and the normal tissues would not be affected. That was an early study. If a perfect balance can be found in the future, the mannose can enhance the chemotherapeutic effect on cancer patients without damaging their overall health". The mannose is a hexose, existing in human blood in minute quantities, it is known that it is supplied not by ingesting food, but by glucose metabolism. CN101347445a composition comprising refined Astragalus polysaccharides for use in strengthening radiation and chemotherapy and reduction toxic and side effects in the treatment of cancer.

CN103830262 provides astragalus polysaccharides for use in strengthening cancer drug therapy such as that of cyclophosphamide.

Although the techniques of targeted drugs and precision medicine are increasingly mature, the drug resistance is still a problem in cancer treatment. If a targeted chemotherapy drug is used in high dose or for a long time, the cancer cells often acquire drug resistance, and survive and keep growing inside the host, leading to chemotherapy drug ineffective cancer treatment eventually. The chemotherapy drug is effective at the initial stage of treatment, at the initial stage of administration, the tumor growth can be inhibited effectively, but after a period of time, the tumor grows again. As the treatment goes on, the drug begins to fail, this phenomenon is medically known as drug resistance. The drug resistance not only makes the cancer recur, but also puts the patient in such a desperate situation that none of drugs is workable, reducing the side effects of chemotherapy drugs and the drug resistance of cancers is an important topic of cancer treatment.

Accordingly, it is very important to develop an effective, safe and reversing drug resistant pharmaceutical composition.

### SUMMARY OF THE INVENTION

In present invention, it is found that an astragalus pharmaceutical composition, which comprising a refined astragalus polysaccharides (hereinafter referred to as r-APS) and a mannose, for the patient receiving chemotherapy, the amount of chemotherapy drug equivalent to the original dose is used in comparison to using the r-APS alone or using the mannose alone, but the effect of chemotherapy drug on reducing the viability of cancer cells can be enhanced, so the therapeutic effect of chemotherapy drug can be enhanced effectively.

The astragalus pharmaceutical composition, which comprising a r-APS and a mannose of the present invention, for the patient with chemotherapy drug resistance, only needs amount of chemotherapy drug equivalent to the original dose, but the effect of chemotherapy drug on reducing the viability of cancer cells can be increased by at least 16%, so even if the patient has been resistant to drug, the therapeutic effect of chemotherapy drug can be enhanced effectively.

In view of the above-mentioned problem, the present invention provides an astragalus pharmaceutical composition for use in a method of enhancing the treatment of a chemotherapy drug in a cancer on inhibiting the viability of a cancer cell, wherein the astragalus pharmaceutical composition comprises a refined astragalus polysaccharides (r-APS) and a

mannose,
wherein the refined astragalus polysaccharides (r-APS) is obtained by the steps of:

(a) Put the astragalus chips in a container, add a pure water to form a solution A;
(b) Heat the solution A formed in step (a) at a time in 2-4 hours, the temperature is 80-100°C;
(c) Collect and concentrate the solution of step (b) to obtain an extraction liquid A;
(d) Put the extraction liquid A of step (c) in a container B, add 30~50% ethanol, stir and precipitate, collect and concentrate the supernatant to obtain a concentrated solution A;
(e) Add 75%~95% ethanol in the concentrated solution A obtained in step (d), stir and precipitate, collect the precipitate A; and
(f) Dehydrate and filter the precipitate A of step (e), dissolve and precipitate with ethanol, centrifuge the precipitate to obtain product A, the product A contains r-APS.

In some embodiments, the chemotherapy drug for the cancer is a targeted chemotherapy drug, the targeted chemotherapy drug is a targeted chemotherapy drug for the corresponding cancer type.
In some embodiments, the cancer comprises colorectal cancer, ovarian cancer, or lung cancer.
In some embodiments, the targeted chemotherapy drug for corresponding cancer type comprises Regorafenib, Olaparib or Tarceva.
In some embodiments, the astragalus pharmaceutical composition can be tablet, pill, granule, powder, capsule, or liquid.
In one embodiment, the cancer comprises a drug resistant cancer.
In one embodiment, the cancer is a solid tumor cancer.
In some embodiments, the astragalus pharmaceutical composition can administer to a subject.
In some embodiments, the astragalus pharmaceutical composition is administered by injection, infusion, intravenous, or oral.
It is to be understood that both the foregoing general description and the following detailed description are exemplary and explanatory only and are not restrictive of the invention.

**BRIEF DESCRIPTION OF THE DRAWINGS**

**Fig. 1A** shows that the results of the r-APS on drug resistant cell line of lung cancer (HCC827GR cells).

**Fig. 1B** shows that the results of the astragalus pharmaceutical composition comprising the r-APS and the mannose on drug resistant cell line of lung cancer (HCC827GR cells).

**Fig. 1C** shows that the results of Iressa as chemotherapy drug for lung cancer on drug resistant cell line of lung cancer (HCC827GR cells).

**Fig. 2A** shows that the results of the r-APS on drug resistant cell line of lung cancer (H1975 cells).

**Fig. 2B** shows that the results of the astragalus pharmaceutical composition comprising the r-APS and the mannose on drug resistant cell line of lung cancer (H1975 cells).

**Fig. 2C** shows that the results of Tarceva as chemotherapy drug for lung cancer on drug resistant cell line of lung cancer (H1975 cells).

**Fig. 3A** shows that the results of the r-APS in combination with Tarceva as chemotherapy drug for lung cancer on drug resistant cells of lung cancer (H1975 cells).

**Fig. 3B** shows that the results of the astragalus pharmaceutical composition comprising the r-APS and the mannose in combination with Tarceva as chemotherapy drug for lung cancer on drug resistant cell line of lung cancer (H1975 cells).

**Fig. 4A** shows that the select results of the 60~70% chemotherapeutic inhibition cultured with cell line of colorectal cancer (HCT116 cells).

**Fig. 4B** shows that the results of cell line of colorectal cancer (HCT116 cells) cultured with the r-APS for 48 hours.

**Fig. 4C** shows that the results of cell line of colorectal cancer (HCT116 cells) cultured with the astragalus pharmaceutical composition comprising the r-APS and the mannose for 48 hours.

**Fig. 4D** shows that the results of cell line of colorectal cancer (HCT116 cells) cultured with the r-APS in combination with Regorafenib as chemotherapy drug for colorectal cancer for 48 hours.

**Fig. 4E** shows that the results of cell line of colorectal cancer (HCT116 cells) cultured with the astragalus pharmaceutical composition comprising the r-APS and the mannose in combination with Regorafenib as chemotherapy drug for colorectal cancer for 48 hours.

**Fig. 5A** shows that the select results of 60~70% chemotherapeutic inhibition cultured with ovarian cancer cell line (OVCAR3 cells).

**Fig. 5B** shows that the results of ovarian cancer cell line (OVCAR cells) cultured with the r-APS for 48 hours.

**Fig. 5C** shows that the results of ovarian cancer cell line (OVCAR cells) cultured with the astragalus pharmaceutical composition comprising the r-APS and the mannose for 48 hours.

**Fig. 5D** shows that the results of ovarian cancer cell line (OVCAR cells) cultured with the r-APS in combination with Olaparib as chemotherapy drug for colorectal cancer for 48 hours.

**Fig. 5E** shows that the result of the astragalus pharmaceutical composition comprising the r-APS and the mannose in combination with Olaparib as chemotherapy drug for colorectal cancer cultured with ovarian cancer cell line (OVCAR cells) for 48 hours.

## DETAILED DESCRIPTION OF THE INVENTION

The "r-APS" in the present invention refers to refined astragalus polysaccharides.

The enhancement of chemotherapy drug in the present invention is enhanced at least 16% of therapeutic effect.

The chemotherapy drug in the present invention comprises targeted chemotherapy drug, and the targeted chemotherapy drug is a targeted chemotherapy drug for corresponding cancer type.

The present invention provides an astragalus pharmaceutical composition for use in a method of enhancing the treatment of a chemotherapy drug in a cancer for inhibiting the viability of a cancer cell, wherein the astragalus pharmaceutical composition comprises a refined astragalus polysaccharides (r-APS) and a mannose, wherein the ratio by weight of the r-APS to the mannose in the astragalus pharmaceutical composition is 1.5~4.5:5.5~8.5. The effective concentration range of the r-APS of the astragalus pharmaceutical composition in present invention is 0~10mg/mL, the effective concentration range of the mannose is 0~10mg/mL. Preferably, the effective concentration of the r-APS is 3mg/mL, the effective concentration of the mannose is 7mg/mL.

Further, the present invention also provides an astragalus pharmaceutical composition for use in a method of enhancing the treatment of a chemotherapy drug in a cancer for treating a drug resistant cancer on inhibiting the viability of a cancer cell, wherein the astragalus pharmaceutical composition comprises a refined astragalus polysaccharides (r-APS) and a mannose, wherein the ratio by weight of the r-APS to the mannose in the astragalus pharmaceutical composition is 1.5~4.5:5.5~8.5. The effective concentration range of r-APS in the astragalus pharmaceutical composition of the present invention is 0~10mg/mL, the effective concentration range of mannose is 0~10mg/mL. Preferably, the effective concentration of r-APS is 3mg/mL, the effective concentration of mannose is 7mg/mL.

Further, the present invention also provides an astragalus pharmaceutical composition for use in a method of enhancing the treatment of a chemotherapy drug in a cancer for inhibiting the proliferation of a tumor cell, wherein the astragalus pharmaceutical composition comprises a refined astragalus polysaccharides (r-APS) and a mannose, wherein the weight ratio of the r-APS to the mannose in the astragalus pharmaceutical composition is 1.5~4.5:5.5~8.5. The effective concentration range of the r-APS in the astragalus pharmaceutical composition of the present invention is 0~10mg/mL, the effective concentration range of the mannose is 0~10mg/mL. Preferably, the effective concentration of the r-APS is 3mg/mL, the effective concentration of the mannose is 7mg/mL.

In some embodiments, the astragalus pharmaceutical composition can administer to a subject.

In some embodiments, the astragalus pharmaceutical composition is administered by injection, infusion, intravenous, or oral.

In some preferred embodiments of the present invention, the method for manufacturing the r-APS in present

invention comprising the steps of:

(a) Put the astragalus chips in a container, add a pure water to form a solution A;
(b) Heat the solution A formed in step (a) at a time in 2-4 hours, the temperature is 80-100°C;
(c) Collect and concentrate the solution of step (b) to obtain an extraction liquid A;
(d) Put the extraction liquid A of step (c) in a container B, add 30~50% ethanol, stir and precipitate, collect and concentrate the supernatant to obtain a concentrated solution A;
(e) Add 75%~95% ethanol in the concentrated solution A obtained in step (d), stir and precipitate, collect the precipitate A; and
(f) Dehydrate and filter the precipitate A of step (e), dissolve and precipitate with ethanol, centrifuge the precipitate to obtain product A, the product A contains r-APS.

Additional specific embodiments of the present invention include, but are not limited to the following:

## EXAMPLE 1

### Influence of the r-APS and the mannose on drug resistant cells of lung cancer

This example discusses the influence of the r-APS and the mannose on the drug resistant cells of lung cancer, there are three test groups, respectively treated as follows:

(1) Test group 1: the different concentrations of the r-APS are 0.25mg/ml, 0.5mg/ml, 1mg/ml, 2mg/ml and 4mg/ml.
(2) Test group 2: the different concentrations of the astragalus pharmaceutical composition comprising the r-APS and the mannose (hereinafter referred to as r-APS+mannose) (the ratio by weight of the r-APS : the mannose is 30%:70%) are 0.625mg/ml, 1.25mg/ml, 2.5mg/ml, 5mg/ml and 10mg/ml.
(3) Test group 3: Iressa (applicable to the HCC827GR cell line) or Tarceva (applicable to the H1975 cell line).

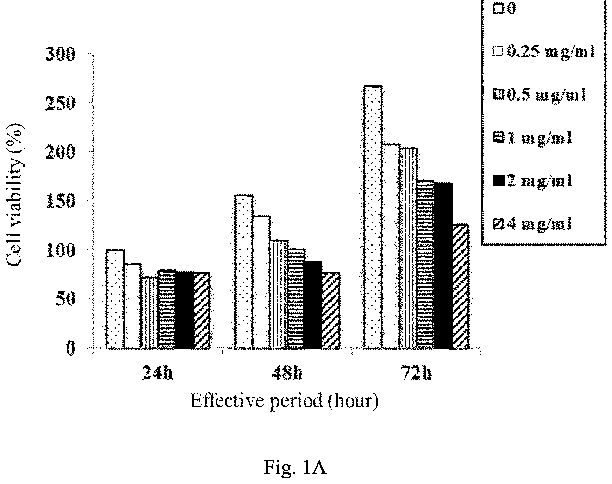

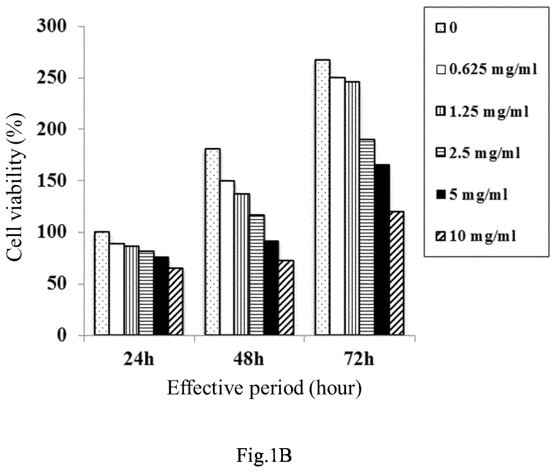

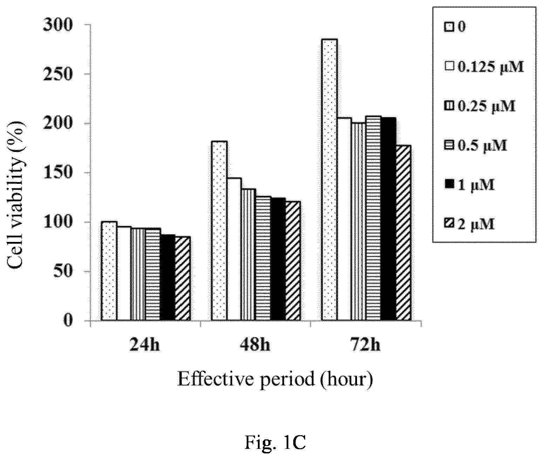

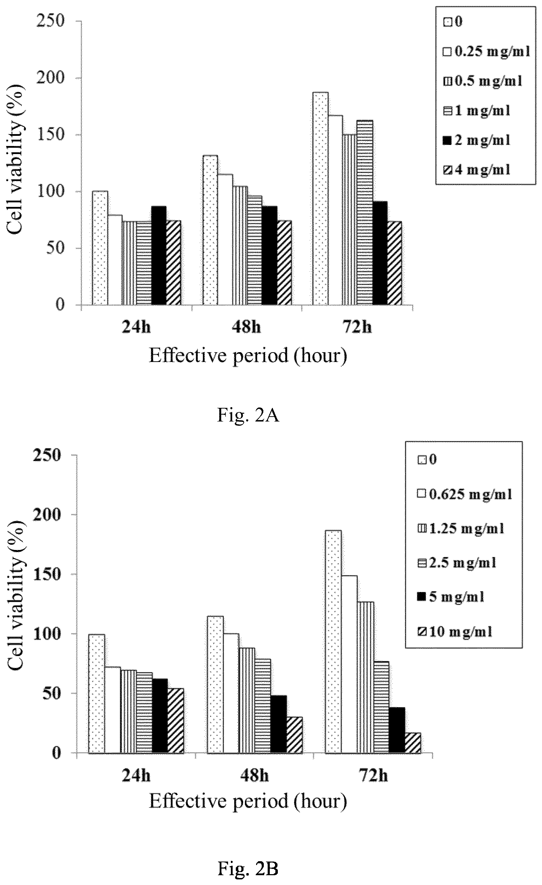

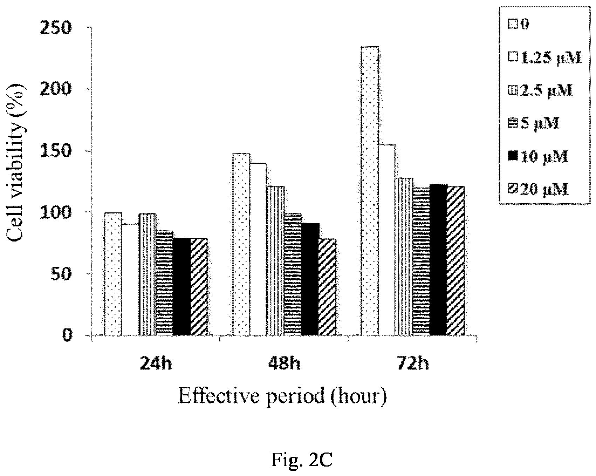

The test group 1, test group 2 and test group 3 are added to the HCC827GR or the H1975 lung cancer cell line respectively, each group incubates for 24 hours, 48 hours and 72 hours respectively, and then the cell viability of the HCC827GR or the H1975 lung cancer cell line is tested. The results are shown in Fig.1A ~ 1C and Fig.2A~2C.

The HCC827GR cell line is resistant to the chemotherapy drug Iressa for treating lung cancer, the result shows that the test group 2 (the r-APS) and test group 3 (the astragalus pharmaceutical composition comprising the r-APS and the mannose (r-APS+mannose)) have better anticancer effect to lung cancer (as shown in Fig.1A~Fig.1C).

The H1975 cell line is a cell line resistant to the drug Tarceva for treating lung cancer, the result shows that the test group 3 (the astragalus pharmaceutical composition comprising the r-APS and the mannose (r-APS+mannose)) has the best anticancer effect (as shown in Fig.2A~2C).

## EXAMPLE 2

### Effect of the astragalus pharmaceutical composition comprising the r-APS and the mannose (r-APS+mannose) in combination with anticancer drug on the drug resistant cells of lung cancer

This example discusses the influence of the astragalus pharmaceutical composition comprising the r-APS and the mannose (r-APS+mannose) on the drug resistant cells of lung cancer, there are three test groups, respectively treated as follows:

(1) Test group 1: the different concentrations of the r-APS are 0.25mg/ml, 0.5mg/ml, 1mg/ml, 2mg/ml and 4mg/ml.
(2) Test group 2: the different concentrations of the astragalus pharmaceutical composition comprising the r-APS and the mannose (r-APS+mannose) (the ratio by weight of the r-APS : the mannose is 30%:70%) are 0.625mg/ml, 1.25mg/ml, 2.5mg/ml, 5mg/ml and 10mg/ml.

Each test group is used with Tarceva (concentrations are 1.25μM, 2.5μM and 5μM) and added to H1975 lung cancer cell line respectively, each group incubates for 24 hours, 48 hours and 72 hours respectively, the cell viability of H1975 lung cancer cell line is measured.

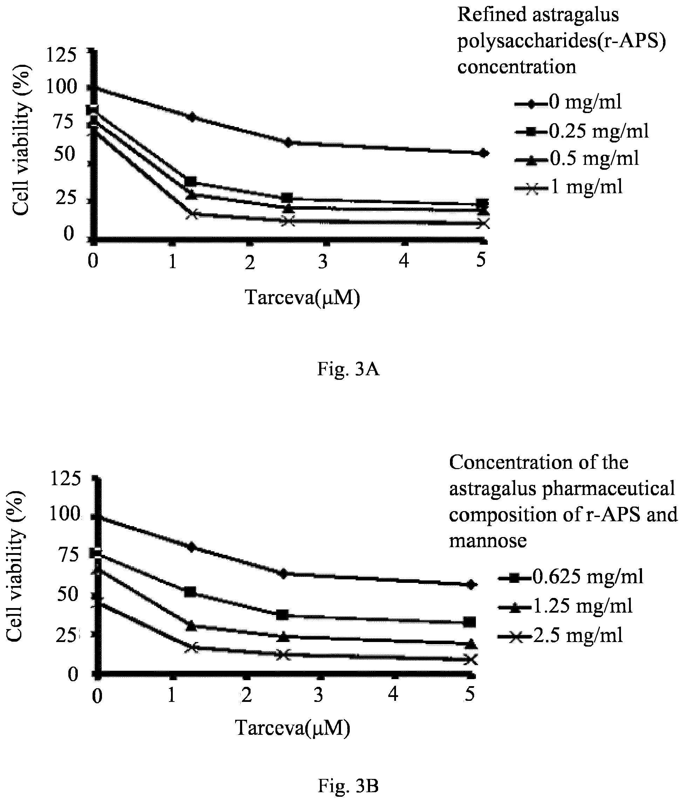

The H1975 cell line is resistant to chemotherapy drug Tarceva, the result shows that the test group 1 (the r-APS) and test group 2 (the astragalus pharmaceutical composition comprising the r-APS and the mannose (r-APS+mannose)) in combination with chemotherapy drug Tarceva have the best anticancer effect (as shown in Figs. 3A~3B), meaning the test group 1 (the r-APS) and test group 2 (the astragalus pharmaceutical composition comprising the r-APS and the

mannose (r-APS+mannose)) in combination with chemotherapy drug Tarceva have the best therapeutic effect on reversing the drug resistance to anticancer drugs of lung cancer.

**EXAMPLE 3**

**Influence of the astragalus pharmaceutical compositionof the invention on colorectal cancer**

Experimental method:

(1) First of all, poisonous dose test (the chemotherapy drug dose for inhibiting the cell growth and leaving 60~70% of growth capability is selected for subsequent test)
(2)The cells are inoculated in 96-well plate and cultured for 24 hours.
(3)Test drugs at different concentrations are added in, the test concentrations of the r-APS are 0, 2 and 4 mg/ml, the test concentrations of the astragalus pharmaceutical composition comprising the r-APS and the mannose (r-APS+mannose) are 0, 5 and 10 mg/ml.
(4) The dose with a poisoning effect on 35-40% of individual cells is selected as chemotherapy drug dose.
(5) The cell viability is tested in 48 and 72 hours respectively after addition.
(6) Experiment on chemotherapeutic synergism of the r-APS and the astragalus pharmaceutical composition comprising the r-APS and the mannose (r-APS+mannose):
(7) The cells are inoculated in 96-well plate and cultured for 24 hours.
(8) The r-APS or the astragalus pharmaceutical composition comprising the r-APS and the mannose (r-APS+mannose) in combination with chemotherapy drug are cultured for 48 hours, the doses of the r-APS and the astragalus pharmaceutical composition comprising the r-APS and the mannose (r-APS+mannose) are selected four poisonous doses for test, and then the cell viability is measured by MTS in 48 hours.

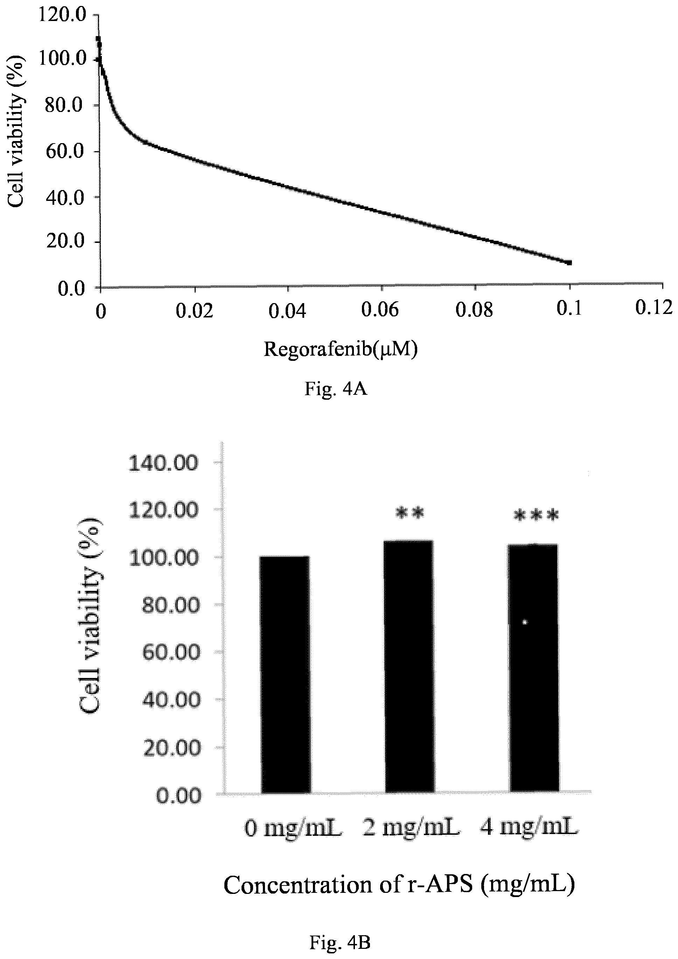

Poisonous dose test result: as shown in Table 1, when the dose of chemotherapy drug Regorafenib is 0.01mM, the cell viability of the cancer cells can be poisoned to 60%, the condition after the cancer patient takes the chemotherapy drug is simulated, the experimental result of chemotherapeutic synergism of the r-APS and the astragalus pharmaceutical composition comprising the r-APS and the mannose (r-APS+mannose) is shown in Fig. 4A.

**Table 1. Cell viability (%) of cancer cells of colorectal cancer poisoned by chemotherapy drug Regorafenib at different concentrations**

| Concentration / Repeat | 0 (mM) | 0.00001 (mM) | 0.0001 (mM) | 0.001 (mM) | 0.01 (mM) | 0.1 (mM) |
|---|---|---|---|---|---|---|
| Repeat 1 | 1.054 | 1.108 | 1.102 | 1.095 | 0.646 | 0.094 |
| Repeat 2 | 0.973 | 1.1 | 1.137 | 0.849 | 0.634 | 0.095 |
| Repeat 3 | 0.991 | 1.1 | 0.985 | 0.906 | 0.637 | 0.089 |
| Mean value (MEAN) | 1.006 | 1.103 | 1.075 | 0.950 | 0.639 | 0.093 |
| Standard deviation (SD) | 0.04 | 0.00 | 0.08 | 0.13 | 0.01 | 0.00 |
| Cell viability (%) | 100.0 | 109.6 | 106.8 | 94.4 | 63.5 | 9.2 |

As shown below in Table 2, the cancer cells of colorectal cancer (HCT116 cells) are added in the r-APS and cultured for 48 hours, the result shows that the growth of cancer cells is promoted slightly when the r-APS is used alone, meaning using the r-APS alone has no significant poisoning effect (as shown in Fig. 4B).

**Table 2. Cell viability (%) of cancer cells of colorectal cancer (HCT116 cells) culture for 48 hours after adding the r-APS at different concentrations.**

| Concentration / Repeat | 0 (mg/ml) | 2 (mg/ml) | 4 (mg/ml) |
|---|---|---|---|
| Repeat 1 | 1.282 | 1.351 | 1.341 |
| Repeat 2 | 1.290 | 1.381 | 1.342 |
| Repeat 3 | 1.294 | 1.374 | 1.347 |
| Mean value (MEAN) | 1.289 | 1.369 | 1.343 |
| Standard deviation (SD) | 0.01 | 0.02 | 0.00 |
| Cell viability (%) | 100.00 | 106.21 | 104.24 |

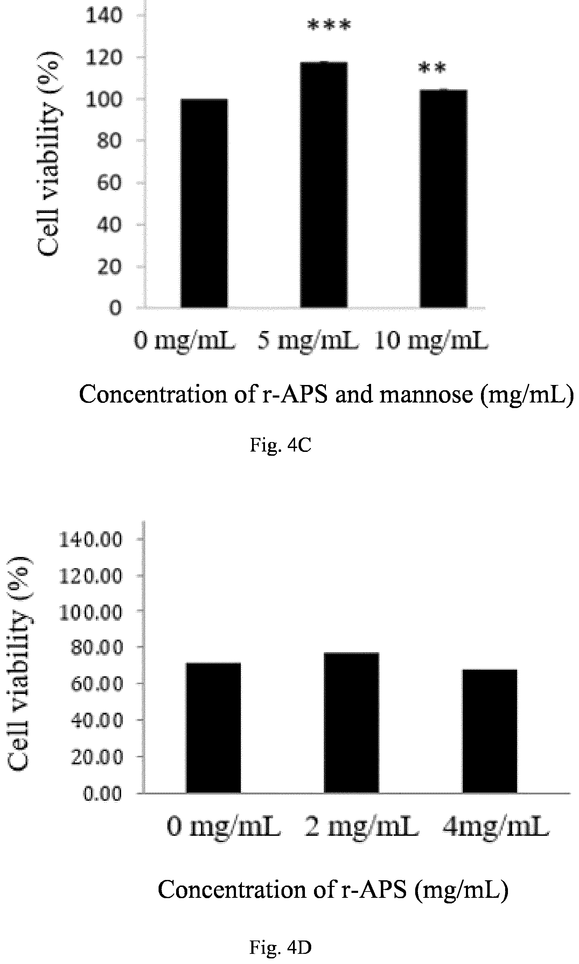

As shown below in Table 3, the HCT116 has been treated with the astragalus pharmaceutical composition comprising the r-APS and the mannose (r-APS+mannose) for 48 hours, the result shows that the astragalus pharmaceutical composition comprising the r-APS and the mannose (r-APS+mannose) promotes the growth of cancer cells on the contrary, meaning the astragalus pharmaceutical composition comprising the r-APS and the mannose (r-APS+mannose) has no significant poisoning effect (as shown in Fig. 4C).

**Table 3, Cell viability (%) of cancer cells of colorectal cancer (HCT116 cells) culture for 48 hours after adding the astragalus pharmaceutical composition comprising the r-APS and the mannose (r-APS+mannose) at different concentrations.**

| Concentration / Repeat | 0 (mg/ml) | 5 (mg/ml) | 10 (mg/ml) |
|---|---|---|---|
| Repeat 1 | 1.282 | 1.524 | 1.328 |
| Repeat 2 | 1.290 | 1.524 | 1.361 |
| Repeat 3 | 1.294 | 1.506 | 1.355 |

| | | | |
|---|---|---|---|
| Mean value (MEAN) | 1.289 | 1.518 | 1.348 |
| Standard deviation (SD) | 0.01 | 0.010 | 0.018 |
| Cell viability (%) | 100 | 117.80 | 104.60 |

As shown below in Table 4, the cancer cells of colorectal cancer (HCT116 cells) have been cultured for 48 hours after adding chemotherapy drug Regorafenib (0.01 mM) and the r-APS, the result shows that the growth of cancer cells is promoted when the effective concentration of the r-APS is 2mg/mL, and the growth of cancer cells is inhibited slightly when the concentration is 4mg/mL, meaning the r-APS in combination with chemotherapy drug has no significant poisoning effect (as shown in Fig. 4D).

**Table 4, Cell viability (%) of cancer cells of colorectal cancer (HCT116 cells) culture for 48 hours after adding chemotherapy drug Regorafenib (0.01 mM) and the r-APS at different concentrations.**

| Concentration / Repeat | 0 (mg/ml) | 2 (mg/ml) | 4 (mg/ml) |
|---|---|---|---|
| Repeat 1 | 0.634 | 0.625 | 0.606 |
| Repeat 2 | 0.646 | 0.710 | 0.614 |
| Repeat 3 | 0.643 | 0.735 | 0.604 |
| Mean value (MEAN) | 0.641 | 0.690 | 0.608 |
| Standard deviation (SD) | 0.01 | 0.06 | 0.01 |
| Cell viability (%) | 71.4 | 76.9 | 67.8 |

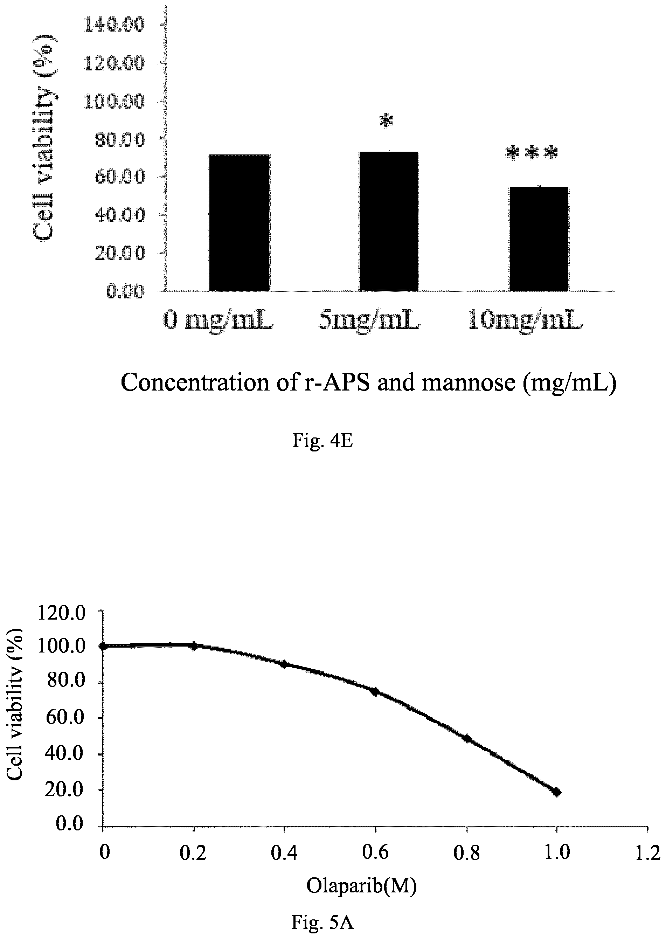

As shown below in Table 5, the HCT116 has been cultured for 48 hours after adding chemotherapy drug Regorafenib (0.01 mM) and the astragalus pharmaceutical composition of the r-APS and the mannose (r-APS+mannose), the result shows that when the effective dose of the astragalus pharmaceutical composition comprising the r-APS and the mannose (r-APS+mannose) is 10mg/mL, the combination of chemotherapy drug can effectively reduce the viability of cancer cells, meaning the astragalus pharmaceutical composition comprising the r-APS and the mannose (r-APS+mannose) in combination with chemotherapy drug has a significant poisoning effect (as shown in Fig. 4E).

**Table 5, Cell viability (%) of cancer cells of colorectal cancer (HCT116 cells) culture for 48 hours after adding chemotherapy drug Regorafenib (0.01 mM) and the astragalus pharmaceutical composition comprising the r-APS and the mannose (r-APS+mannose).**

| Concentration / Repeat | 0 (mg/ml) | 5 (mg/ml) | 10 (mg/ml) |
|---|---|---|---|
| Repeat 1 | 0.634 | 0.661 | 0.485 |
| Repeat 2 | 0.646 | 0.670 | 0.495 |
| Repeat 3 | 0.643 | 0.653 | 0.497 |
| Mean value (MEAN) | 0.641 | 0.661 | 0.492 |
| Standard deviation (SD) | 0.006 | 0.01 | 0.01 |
| Cell viability (%) | 71.43 | 73.70 | 54.90 |

The experimental results show that the astragalus pharmaceutical composition comprising the r-APS and the mannose (r-APS+mannose) in combination with chemotherapy drug of the present invention can further reduce the viability of cancer cells of colorectal cancer by 16% compared with only using drug, so the therapeutic effect of chemotherapy drug can be enhanced effectively.

**EXAMPLE 4**

**Influence of the astragalus pharmaceutical composition of the present invention on ovarian cancer**

Experimental method:

(1) Poisonous dose test (the chemotherapy drug dose for inhibiting the cell growth and leaving 60~70% of growth capability is selected for subsequent test).
(2) The cells are inoculated in 96-well plate and cultured for 24 hours.
(3) Test drugs at different concentrations are added in, the test concentrations of the r-APS are 0, 2 and 4 mg/ml, the test concentrations of the astragalus pharmaceutical composition comprising the r-APS and the mannose (r-APS+mannose) are 0, 5 and 10 mg/ml.
(4) The dose with a poisoning effect on 35-40% of individual cells is selected as chemotherapy drug dose.
(5) The cell viability is tested in 48 and 72 hours respectively after addition.
(6) Experiment on chemotherapeutic synergism of the r-APS and the astragalus pharmaceutical composition comprising the r-APS and the mannose (r-APS+mannose).
(7) The cells are inoculated in 96-well plate and cultured for 24 hours.
(8) The r-APS or the astragalus pharmaceutical composition comprising the r-APS and the mannose (r-APS+man-

nose) in combination with chemotherapy drug are cultured for 48 hours, the dose of the r-APS and the astragalus pharmaceutical composition comprising the r-APS and the mannose (r-APS+mannose) is three poisonous doses for test, the cell viability is measured by MTS in 48 hours.

The poisonous dose test result is shown below in Table 6, when the dose of chemotherapy drug Olaparib is 0.7M, the cell viability of cancer cells can be poisoned to 60%, the condition after the cancer patient takes the chemotherapy drug is simulated, the experimental result of chemotherapeutic synergism of the r-APS and the astragalus pharmaceutical composition comprising the r-APS and the mannose (r-APS+mannose) is shown in Fig. 5A.

**Table 6, Cell viability (%) of ovarian cancer cell line (OVCAR cells) poisoned by chemotherapy drug Olaparib at different concentrations**

| Concentration / Repeat | 0 M | 0.2 M | 0.4 M | 0.6 M | 0.8 M | 1 M |
|---|---|---|---|---|---|---|
| Repeat 1 | 0.984 | 0.984 | 0.895 | 0.752 | 0.531 | 0.214 |
| Repeat 2 | 0.982 | 0.991 | 0.871 | 0.738 | 0.413 | 0.152 |
| Repeat 3 | 0.969 | 0.976 | 0.885 | 0.715 | 0.486 | 0.184 |
| Mean value (MEAN) | 0.978 | 0.984 | 0.884 | 0.735 | 0.477 | 0.183 |
| Standard deviation (SD) | 0.008 | 0.008 | 0.012 | 0.019 | 0.060 | 0.031 |
| Cell viability (%) | 100.00 | 100.55 | 90.32 | 75.13 | 48.72 | 18.74 |

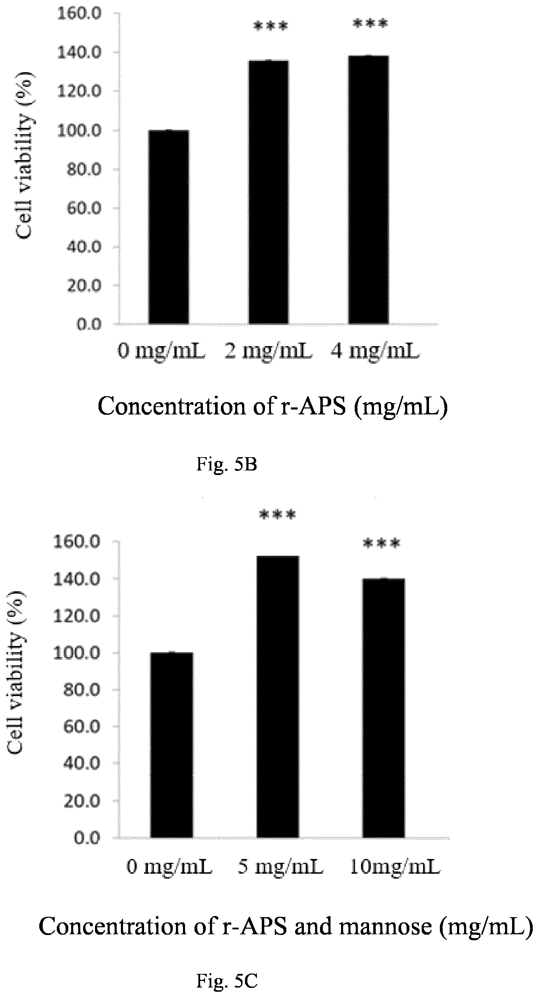

The experimental result of chemotherapeutic synergism of the r-APS and the astragalus pharmaceutical composition comprising the r-APS and the mannose (r-APS+mannose) is shown in Table 7, the ovarian cancer cell line (OVCAR cells) is cultured with the r-APS for 48 hours, the result shows that using the r-APS alone promotes the growth of cancer cells on the contrary, meaning using the r-APS alone has no significant poisoning effect (as shown in Fig. 5B)

**Table 7, Cell viability (%) of ovarian cancer cell line (OVCAR cells) culture for 48 hours after adding the r-APS at different concentrations.**

| Concentration / Repeat | 0 (mg/ml) | 2 (mg/ml) | 4 (mg/ml) |
|---|---|---|---|
| Repeat 1 | 0.874 | 1.165 | 1.198 |
| Repeat 2 | 0.873 | 1.215 | 1.237 |
| Repeat 3 | 0.877 | 1.183 | 1.193 |

| | | | |
|---|---|---|---|
| Mean value (MEAN) | 0.875 | 1.188 | 1.209 |
| Standard deviation (SD) | 0.002 | 0.025 | 0.024 |
| Cell viability (%) | 100.0 | 135.8 | 138.3 |

As shown below in Table 8, the ovarian cancer cell line (OVCAR cells) has been cultured for 48 hours after adding the astragalus pharmaceutical composition comprising the r-APS and the mannose (r-APS+mannose), the result shows that the astragalus pharmaceutical composition comprising the r-APS and the mannose (r-APS+mannose) promotes the growth of cancer cells, meaning using the astragalus pharmaceutical composition comprising the r-APS and the mannose (r-APS+mannose) alone has no significant poisoning effect (as shown in Fig. 5C).

**Table 8, Cell viability (%) of ovarian cancer cell line (OVCAR cells) culture for 48 hours after adding the astragalus pharmaceutical composition comprising the r-APS and the mannose (r-APS+mannose) at different concentrations**

| Concentration / Repeat | 0 (mg/ml) | 5 (mg/ml) | 10 (mg/ml) |
|---|---|---|---|
| Repeat 1 | 0.874 | 1.318 | 1.211 |
| Repeat 2 | 0.877 | 1.351 | 1.251 |
| Repeat 3 | 0.877 | 1.323 | 1.213 |
| Mean value (MEAN) | 0.876 | 1.331 | 1.225 |
| Standard deviation (SD) | 0.002 | 0.018 | 0.023 |
| Cell viability (%) | 100.2 | 152.1 | 140.1 |

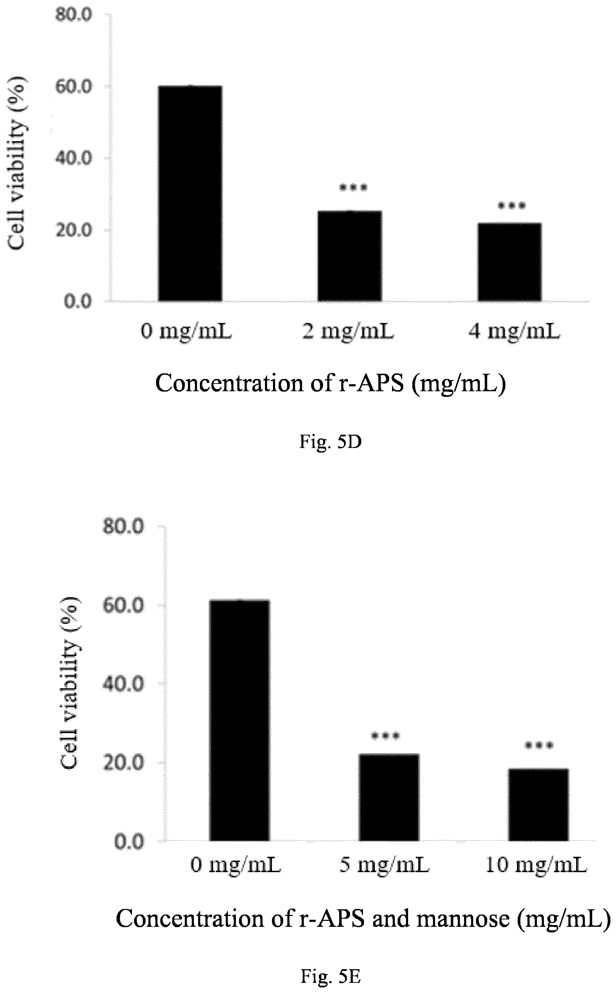

As shown in Table 9, the ovarian cancer cell line (OVCAR cells) has been cultured with Olaparib (0.7 M) and the r-APS for 48 hours, the result shows that the r-APS in combination with chemotherapy drug Olaparib can effectively inhibit the viability of ovarian cancer cell line (OVCAR cells), so the r-APS in combination with chemotherapy drug has a significant poisoning effect (as shown in Fig. 5D).

**Table 9, Cell viability (%) of ovarian cancer cell line (OVCAR cells) culture for 48 hours after adding Olaparib (0.7 M) and the r-APS.**

| Repeat \ Concentration | 0 (mg/ml) | 2 (mg/ml) | 4 (mg/ml) |
|---|---|---|---|
| Repeat 1 | 0.598 | 0.252 | 0.224 |
| Repeat 2 | 0.584 | 0.244 | 0.216 |
| Repeat 3 | 0.602 | 0.255 | 0.210 |
| Mean value (MEAN) | 0.595 | 0.250 | 0.217 |
| Standard deviation (SD) | 0.009 | 0.006 | 0.006 |
| Cell viability (%) | 60.189 | 25.337 | 21.930 |

As shown in Table 10, the ovarian cancer cell line (OVCAR cells) has been cultured with Olaparib (0.7M) and the astragalus pharmaceutical composition comprising the r-APS and the mannose (r-APS+mannose) for 48 hours, the result shows that the astragalus pharmaceutical composition comprising the r-APS and the mannose (r-APS+mannose) in combination with chemotherapy drug can effectively inhibit the cell viability of ovarian cancer cell line (OVCAR cells), so the astragalus pharmaceutical composition comprising the r-APS and the mannose (r-APS+mannose) in combination with chemotherapy drug has a significant poisoning effect (as shown in Fig. 5E).

**Table 10, Cell viability (%) of ovarian cancer cell line (OVCAR cells) culture for**

**48 hours after adding Olaparib (0.7 M) and the astragalus pharmaceutical composition comprising the r-APS and the mannose (r-APS+mannose)**

| Repeat \ Concentration | 0 (mg/ml) | 5 (mg/ml) | 10 (mg/ml) |
|---|---|---|---|
| Repeat 1 | 0.598 | 0.220 | 0.183 |
| Repeat 2 | 0.584 | 0.205 | 0.185 |
| Repeat 3 | 0.632 | 0.227 | 0.175 |
| Mean value (MEAN) | 0.605 | 0.217 | 0.181 |
| Standard deviation (SD) | 0.025 | 0.011 | 0.005 |
| Cell viability (%) | 61.4 | 22.1 | 18.4 |

The experimental results show that the astragalus pharmaceutical composition comprising the r-APS and the mannose (r-APS+mannose) in combination with chemotherapy drug of the present invention can further reduce the viability of cancer cells of colorectal cancer by 43% compared with only using drug, so the therapeutic effect of chemotherapy drug can be enhanced effectively.

To sum up, the astragalus pharmaceutical composition comprising the r-APS and the mannose (r-APS+mannose) in combination with chemotherapy drug of the present invention can reduce the cell viability of cancer cells by at least 16%, so the ability of chemotherapy drug to reduce the cell viability of cancer cells can be enhanced effectively, and the proliferation of tumor cells can be inhibited effectively, the chemotherapeutic effect is stronger than only using r-APS, even on drug resistant cancers, the therapeutic effect of chemotherapy drug can be maintained or enhanced.

## Claims

1. An astragalus pharmaceutical composition for use in a method of enhancing the treatment of a chemotherapy drug in a cancer on inhibiting the viability of a cancer cell,

   wherein the astragalus pharmaceutical composition comprises a refined astragalus polysaccharides (r-APS) and a mannose,
   wherein the refined astragalus polysaccharides (r-APS) is obtained by the steps of:

   (a) Put the astragalus chips in a container, add a pure water to form a solution A;
   (b) Heat the solution A formed in step (a) at a time in 2-4 hours, the temperature is 80-100°C;
   (c) Collect and concentrate the solution of step (b) to obtain an extraction liquid A;
   (d) Put the extraction liquid A of step (c) in a container B, add 30~50% ethanol, stir and precipitate, collect and concentrate the supernatant to obtain a concentrated solution A;
   (e) Add 75%~95% ethanol in the concentrated solution A obtained in step (d), stir and precipitate, collect the precipitate A; and
   (f) Dehydrate and filter the precipitate A of step (e), dissolve and precipitate with ethanol, centrifuge the precipitate to obtain product A, the product A contains r-APS.

2. The astragalus pharmaceutical composition for use according to claim 1, wherein the cancer comprises a drug

resistant cancer.

3. The astragalus pharmaceutical composition for use according to claim 1, wherein the treatment effect comprises inhibiting the viability of a cancer cell.

4. The astragalus pharmaceutical composition for use according to claim 2, wherein the cancer is solid tumor cancer.

5. The astragalus pharmaceutical composition for use according to claim 1, wherein the cancer comprises colorectal cancer, ovarian cancer, or lung cancer.

6. The astragalus pharmaceutical composition for use according to claim 1, wherein the targeted chemotherapy drug for the corresponding cancer type comprises Regorafenib, Olaparib or Tarceva.

7. The astragalus pharmaceutical composition for use according to claim 1-7, wherein the form of the astragalus pharmaceutical composition can be tablet, pill, granule, powder, capsule, or liquid.

**Patentansprüche**

1. Pharmazeutische Astragalus-Zusammensetzung zur Verwendung in einem Verfahren zur Verbesserung der Behandlung mit einem Chemotherapeutikum bei einer Krebserkrankung durch Hemmung der Lebensfähigkeit von Krebszellen, wobei die pharmazeutische Astragalus-Zusammensetzung raffinierte Astragalus-Polysaccharide (r-APS) und Mannose umfasst,
wobei die raffinierten Astragalus-Polysaccharide (r-APS) durch folgende Schritte gewonnen werden:

(a) Einbringen der Astragalus-Schnitzel in einen Behälter, Zugabe von reinem Wasser zur Bildung einer Lösung A;
(b) Erhitzen der in Schritt (a) gebildeten Lösung A über einen Zeitraum von 2-4 Stunden auf eine Temperatur von 80-100 °C;
(c) Auffangen und Konzentrieren der Lösung aus Schritt (b), um eine Extraktionsflüssigkeit A zu erhalten;
(d) Einbringen der Extraktionsflüssigkeit A aus Schritt (c) in einen Behälter B, Zugabe von 30-50 % Ethanol, Rühren und Ausfällen; Sammeln und Konzentrieren des Überstands, um eine konzentrierte Lösung A zu erhalten;
(e) Zugabe von 75-95 % Ethanol zu der in Schritt (d) erhaltenen konzentrierten Lösung A, Rühren und Ausfällen; Sammeln des Niederschlags A; und
(f) Entwässern und Filtrieren des Niederschlags A aus Schritt (e), Auflösen und Ausfällen mit Ethanol, Zentrifugieren des Niederschlags, um das Produkt A zu erhalten, wobei das Produkt A r-APS enthält.

2. Pharmazeutische Astragalus-Zusammensetzung zur Verwendung gemäß Anspruch 1, wobei der Krebs einen arzneimittelresistenten Krebs umfasst.

3. Pharmazeutische Astragalus-Zusammensetzung zur Verwendung gemäß Anspruch 1, wobei die Behandlungswirkung die Hemmung der Lebensfähigkeit einer Krebszelle umfasst.

4. Pharmazeutische Astragalus-Zusammensetzung zur Verwendung gemäß Anspruch 2, wobei es sich bei dem Krebs um einen soliden Tumor handelt.

5. Pharmazeutische Astragalus-Zusammensetzung zur Verwendung gemäß Anspruch 1, wobei der Krebs Kolorektalkrebs, Eierstockkrebs oder Lungenkrebs umfasst.

6. Pharmazeutische Astragalus-Zusammensetzung zur Verwendung gemäß Anspruch 1, wobei das zielgerichtete Chemotherapeutikum für die entsprechende Krebsart Regorafenib, Olaparib oder Tarceva umfasst.

7. Pharmazeutische Astragalus-Zusammensetzung zur Verwendung gemäß den Ansprüchen 1-7, wobei die Darreichungsform der pharmazeutischen Astragalus-Zusammensetzung eine Tablette, eine Pille, Granulat, Pulver, eine Kapsel oder eine Flüssigkeit sein kann.

## Revendications

1. Composition pharmaceutique d'astragale pour une utilisation dans un procédé d'amélioration du traitement d'un médicament de chimiothérapie dans un cancer sur l'inhibition de la viabilité d'une cellule cancéreuse,

    dans laquelle la composition pharmaceutique d'astragale comprend des polysaccharides d'astragale raffinés (r-APS) et un mannose,
    dans laquelle les polysaccharides d'astragale raffinés (r-APS) sont obtenus par les étapes consistant à :

    (a) placer les copeaux d'astragale dans un récipient, ajouter de l'eau pure pour former une solution A ;
    (b) chauffer la solution A formée à l'étape (a) en 2 à 4 heures, à une température de 80 à 100°C ;
    (c) collecter et concentrer la solution de l'étape b) pour obtenir un liquide d'extraction A ;
    (d) placer le liquide d'extraction A de l'étape c) dans un récipient B, ajouter de l'éthanol à 30-50%, agiter et précipiter, collecter et concentrer le surnageant pour obtenir une solution concentrée A ;
    (e) ajouter de l'éthanol à 75%-95% à la solution concentrée A obtenue à l'étape (d), agiter et précipiter, collecter le précipité A ; et
    (f) déshydrater et filtrer le précipité A de l'étape e), dissoudre et précipiter avec de l'éthanol, centrifuger le précipité pour obtenir le produit A, le produit A contient des r-APS.

2. Composition pharmaceutique d'astragale selon la revendication 1, dans laquelle le cancer est un cancer résistant aux médicaments.

3. Composition pharmaceutique d'astragale pour une utilisation selon la revendication 1, dans laquelle l'effet thérapeutique comprend l'inhibition de la viabilité d'une cellule cancéreuse.

4. Composition pharmaceutique d'astragale pour une utilisation selon la revendication 2, dans laquelle le cancer est un cancer à tumeur solide.

5. Composition pharmaceutique d'astragale pour une utilisation selon la revendication 1, dans laquelle le cancer comprend le cancer colorectal, le cancer de l'ovaire ou le cancer du poumon.

6. Composition pharmaceutique d'astragale pour une utilisation selon la revendication 1, dans laquelle le médicament de chimiothérapie ciblée pour le type de cancer correspondant comprend le Regorafenib, l'Olaparib ou le Tarceva.

7. Composition pharmaceutique d'astragale pour une utilisation selon les revendications 1 à 7, dans laquelle la composition pharmaceutique d'astragale peut se présenter sous la forme d'un comprimé, d'une pilule, d'un granule, d'une poudre, d'une capsule ou d'un liquide.

0
0.25 mg/ml
0.5 mg/ml
1 mg/ml
2 mg/ml
4 mg/ml
300
250
200
150
100
50
0
Cell viability (%)
24h
48h
72h
Effective period (hour)

Fig. 1A

300
250
200
150
100
50
0
Cell viability (%)
24h
48h
72h
Effective period (hour)
0
0.625 mg/ml
1.25 mg/ml
2.5 mg/ml
5 mg/ml
10 mg/ml

Fig.1B

300
250
200
150
100
50
0
Cell viability (%)
24h
48h
72h
Effective period (hour)
0
0.125 µM
0.25 µM
0.5 µM
1 µM
2 µM

Fig. 1C

250
200
150
100
50
0
Cell viability (%)
24h
48h
72h
Effective period (hour)
0
0.25 mg/ml
0.5 mg/ml
1 mg/ml
2 mg/ml
4 mg/ml

Fig. 2A

250
200
150
100
50
0
Cell viability (%)
24h
48h
72h
Effective period (hour)
0
0.625 mg/ml
1.25 mg/ml
2.5 mg/ml
5 mg/ml
10 mg/ml

Fig. 2B

250
200
150
100
50
0
Cell viability (%)
24h
48h
72h
Effective period (hour)
0
1.25 µM
2.5 µM
5 µM
10 µM
20 µM


Fig. 2C

Refined astragalus polysaccharides(r-APS) concentration
0 mg/ml
0.25 mg/ml
0.5 mg/ml
1 mg/ml
Cell viability (%)
125
100
75
50
25
0
0
1
2
3
4
5
Tarceva(μM)

Fig. 3A

Concentration of the astragalus pharmaceutical composition of r-APS and mannose
0.625 mg/ml
1.25 mg/ml
2.5 mg/ml
Cell viability (%)
125
100
75
50
25
0
0
1
2
3
4
5
Tarceva(μM)

Fig. 3B

120.0
100.0
80.0
60.0
40.0
20.0
0.0
Cell viability (%)
0
0.02
0.04
0.06
0.08
0.1
0.12
Regorafenib(μM)

Fig. 4A

140.00
120.00
100.00
80.00
60.00
40.00
20.00
0.00
Cell viability (%)
**
***
0 mg/mL
2 mg/mL
4 mg/mL
Concentration of r-APS (mg/mL)

Fig. 4B

Cell viability (%)
140
120
100
80
60
40
20
0
***
**
0 mg/mL
5 mg/mL
10 mg/mL
Concentration of r-APS and mannose (mg/mL)


Fig. 4C

Cell viability (%)
140.00
120.00
100.00
80.00
60.00
40.00
20.00
0.00
0 mg/mL
2 mg/mL
4mg/mL
Concentration of r-APS (mg/mL)


Fig. 4D

Cell viability (%)
140.00
120.00
100.00
80.00
60.00
40.00
20.00
0.00
*
***
0 mg/mL
5mg/mL
10mg/mL
Concentration of r-APS and mannose (mg/mL)

Fig. 4E

Cell viability (%)
120.0
100.0
80.0
60.0
40.0
20.0
0.0
0
0.2
0.4
0.6
0.8
1.0
1.2
Olaparib(M)

Fig. 5A

160.0
140.0
120.0
100.0
80.0
60.0
40.0
20.0
0.0
Cell viability (%)
***
***
0 mg/mL
2 mg/mL
4 mg/mL
Concentration of r-APS (mg/mL)

Fig. 5B

160.0
140.0
120.0
100.0
80.0
60.0
40.0
20.0
0.0
Cell viability (%)
***
***
0 mg/mL
5 mg/mL
10mg/mL
Concentration of r-APS and mannose (mg/mL)

Fig. 5C

80.0
60.0
40.0
20.0
0.0
Cell viability (%)
***
***
0 mg/mL
2 mg/mL
4 mg/mL
Concentration of r-APS (mg/mL)

Fig. 5D

80.0
60.0
40.0
20.0
0.0
Cell viability (%)
***
***
0 mg/mL
5 mg/mL
10 mg/mL
Concentration of r-APS and mannose (mg/mL)

Fig. 5E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5268467 A
- CN 101347445
- CN 103830262